Europäisches Patentamt

European Patent Office

Office européen des brevets

(19)

(11) Veröffentlichungsnummer: **0 148 391**
**B1**

(12) **EUROPÄISCHE PATENTSCHRIFT**

(45) Veröffentlichungstag der Patentschrift: **06.06.90**

(21) Anmeldenummer: **84114307.6**

(22) Anmeldetag: **27.11.84**

(51) Int. Cl.⁵: **A 61 L 15/22** // A61L15/24, A61L15/44

(54) **Wirkstoffabgabesysteme.**

(30) Priorität: **10.12.83 DE 3344691**

(43) Veröffentlichungstag der Anmeldung:
**17.07.85 Patentblatt 85/29**

(45) Bekanntmachung des Hinweises auf die
Patenterteilung:
**06.06.90 Patentblatt 90/23**

(84) Benannte Vertragsstaaten:
**AT BE CH DE FR GB IT LI NL SE**

(56) Entgegenhaltungen:
**EP-A-0 033 615**
**DE-A-3 315 245**
**GB-A-2 045 618**
**GB-A-2 073 588**

(73) Patentinhaber: **BAYER AG**
**D-5090 Leverkusen 1 Bayerwerk (DE)**

(72) Erfinder: **VON Bittera, Miklos**
**Max-Scheler-Strasse 7**
**D-5090 Leverkusen 3 (DE)**
Erfinder: **Dhein, Rolf, Dr.**
**Deswatinesstrasse 30**
**D-4150 Krefeld (DE)**
Erfinder: **Meyer, Rolf-Volker, Dr.**
**Buchheimer Strasse 23**
**D-4150 Krefeld (DE)**
Erfinder: **Rupp, Roland, Dr.**
**Solinger Strasse 18**
**D-5653 Leichlingen 2 (DE)**

**Die Akte enthält technische Angaben, die nach dem Eingang der Anmeldung eingereicht wurden und die nicht in dieser Patentschrift enthalten sind.**

Courier Press, Leamington Spa, England.

**Beschreibung**

Die Erfindung betrifft ein medizinisches Pflaster zur transdermalen Verabreichung von Etofenamat über einen längeren Zeitraum.

In der US—A—4 031 894 werden medizinische Pflaster beschrieben, die ein Reservoir aus Mineralöl und Polyisobuten besitzen. Das Polyisobuten stellt eine Mischung aus Komponenten verschiedener Molgewichte dar, nämlich PIB von M.G. 35.000 und 1.000.000—1.500.000.

Dieses Pflaster ist nur für Wirkstoffe, die in sehr geringen Dosen appliziert werden müssen, geeignet. In der US-PS wird Scopolamin genannt.

Die britische Offenlegungsschrift GB—A—2 073 588 betrifft eine Haftklebeschicht von Pflastern zur Applikation von Nitroglyzerin. Als Polymerbasis werden Naturkautschuk oder Naturkautschuk-Verschnitte eingesetzt. Eine Beimischung z.B. von niedrig-molekularem Polyisobutylen (20%) dient hier zur Verbesserung der Kautschukeigenschaften bezüglich Flexibilität und Gasundurchlässigkeit. Naturkautschuke dieser Art sind für Etofenamat nicht geeignet.

In der GB—A—2 045 618 werden Pflaster auf Basis von Styrol/Isopren/Styrol-Blockcopolymeren zur Applikation von Antiphlogistika beschrieben. Als Beispiel wird u.a. Etofenamat genannt. Die Freisetzungsrate dieser Pflaster ist jedoch unbefriedigend.

In der DE—A—3 315 245 bzw. EP 144 486 werden Pflaster, bestehend aus einem Wirkstoffreservoir und einer Haftklebschicht offenbart. Das Wirkstoffreservoir besteht aus mehreren Schichten auf Basis von Polyisobutylen, wobei die einzelnen Schichten steigende Wirkstoffkonzentration mit zunehmenden Abstand von der Haut haben. Als geeignete Wirkstoffe werden in einer Aufzählung allgemein auch Antirheumatika genannt.

Die EP—A—33 615 betrifft Applikationssysteme für Nitroglyzerin in Form von polymeren Gelen. Ein solches Gel auf Basis von Polyisobutylen wird beispielsweise erhalten durch hohe Anteile von flüssigen Beimischungen wie niedrigmolekularem PIB und Paraffinöl zu einem höhermolekularen Polyisobutylen Basispolymeren. Gele dieser Art sind zur Applikation von Etofenamat nicht geeignet.

Die FR—A—2 497 457 offenbart Pflaster zur Applikation von Nitraten (ISDN, PETN), die bei einer befriedigenden Freisetzungsrate für die Nitrate einen guten Tragkomfort gewährleisten. Dies wird erreicht durch Verwendung von Polymeren, die eine bestimmte Glasübergangstemperatur haben.

Diese Bedingung wird beispielsweise von Acrylpolymeren, Isopren-Kautschuken sowie von PIB-Gemischen mit niederem und hohem Molekulargewicht erfüllt. Diese Polymeren sind jedoch bei der lösung der unterstehend geschilderden Aufgabestellung ausefriedigend.

Es ist daher eine Aufgabe der vorliegenden Erfindung, medizinsche Pflaster zu entwickeln, mit deren Hilfe über einen länger Zeitraum geregelte größere, therapeutisch wirksame Mengen des Wirkstoffs Etofenamat über die Haut verabreicht werden könne. Diese Pflaster sollen mit der Haut verträglich sein und mit ihrer Hilfe soll es möglich sein, hohe therapeutisch wirksame Dosen des Wirkstoffs Etofenamat zu verabreichen.

Bekannte Wirkstoffabgabesysteme, wie z.B. Gele, Salben, Pflaster, u.ä. erlauben nur eine begrenzte Wirkstoffresorption durch die Haut. Die Resorption hängt von der Grundlage und den Wirkstoffeigenschaften ab.

Es wurde nun gefunden, daß medizinische Pflaster, die den Wirkstoff Etofenamat in einer Reservoirschicht, die als Polymer Polyisobutylen mit einer definierten Molmassenverteilung enthalten, für diesen Zweck besonders gut geeignet sind.

Die Erfindung betrifft ein medizinisches Pflaster zur transdermalen Verabreichung von Antiphlogistika, bestehend aus einer Deckschicht, einer abziehbaren Schutzschicht und einer den Wirkstoff, Schleppmittel und Harze enthaltenden, einlagigen Reservoirschicht auf Basis von Polymeren dadurch gekennzeichnet, daß die Reservoirschicht als Polymere Polyisobutylene mit einer Molmassen-Verteilung $M_z/M_n$ von 1,5 bis 3,5 und eine mittlere Viskosität/Molmasse von 30 000 bis 4 000 000 g/mol enthält und 1 bis 30 Gew.-% des Wirkstoffs Etofenamats enthält.

Vorzugsweise besteht die Wirkstoffreservoirschicht aus 30 bis 60 Gew.-% Polymerkomponente, 30 bis 60 Gew.-% Schleppmittel und 2 bis 40 Gew.-% eines klebrigmachenden Harzes, wobei sich die drei Komponenten zu 100 Gew.-% ergänzen.

Als Polyisobutylene im Sinne der Erfindung werden Polyisobutylene verstanden, die vorzugsweise eine Molmassen-Verteilung $M_w/M_n$ von 2,0 bis 3,0 haben. Bevorzugt haben die Polyisobutylene eine mittlere Viskosität/Molmasse 50 000 bis 1 000 000 g/mol, besonders bevorzugt von 80 000 bis 500 000 g/mol. Die mittlere Viskosität läßt sich in bekannter Weise bestimmen gemäß Polymer-Handbook, J. Brandrup und F. H. Immergut, Wiley & Sons, New York, 1975, Kap. IV, S. 35.

Diese Polyisobutylene sind seit langem bekannt und können z.B. gemäß US—A—2 203 873 oder gemäß DR—B—704,038 mit sauren Katalysatoren hergestellt werden.

Unter *Schleppmittel* im Sinne der vorliegenden Erfindung werden Öle, Fettsäureester, Triglyceride, Alkohole und/oder Fettsäuren verstanden.

Unter Ölen im Sinne der vorliegenden Erfindung werden hochsiedende, aliphatische, araliphatische und/oder aromatische Kohlenwasserstoffe verstanden, vorzugsweise Paraffinöl, Purcellinöl, Perhydrosqalen- und Lösungen von mikrokristallinen Wachsen in den Ölen, Mineralöle, bevorzugt Öle, deren Siedebereich zwischen 150°C und 400°C liegt; ferner ungesättigte Kohlenwasserstoffe mit mindestens 16 C-Atomen wie z.B. Oligomere von Monoolefinen wie Tetraisobutylen, Pentaisobutylen, Hexaisobutylen oder auch flüssige Polymerisate aus Dien(Monoen)-(Co)-Polymerisaten. Beispiele für flüssige Polymerisate aus konjugierten Dienen sind solche aus Butadien, Isopren, 1,3-Pentadien, 2,3-Dimethylbutadien, Copolymerisate verschiedener Diene sowie auch flüssige Copolymerisate aus einem konjugierten Diolefin und geringen Mengen von Monoolefinen wie z.B. Buten-1, Isobuten, Hexen-1, Octen-1, Styrol mit MG von 400 bis 6000, vorzugsweise 800 bis 3000 sowie Idozahlen von 200 bis 500 und Viskositäten von 100 bis 10.000 cP bei 50°C.

Besonders bevorzugt sind flüssige Polybutadien-Polymerisate, die zu mindestens 90% 1,4-verknüpft sind, deren Anteil an cis-Doppelbindungen mehr als 60% beträgt und deren Molmassen 1000 bis 4000 betragen.

Unter Ölen werden auch Silikonöle verschiedener Viskositäten, vorzugsweise mit mittleren Molgewichten von 312 bis 15.000, besonders bevorzugt Polydimethylsiloxane, verstanden.

Unter Fettsäureestern werden solche verstanden, die mindestens 12 C-Atomen, vorzugsweise 15 bis 46 C-Atome, besonders bevorzugt 16 bis 36 C-Atome enthalten. Insbesondere werden darunter verstanden: Ethylstearat, Laurinsäurehexylester, Dipropylen-glykolpelargonat, Palmitinsäurecetylester, Isopropylmyristat, Isopropylpalmitat, Capryl/Caprinsäureester von gesättigten Fettalkoholen der Kettenlänge $C_{12}$—$C_{18}$, Isopropylstearat, Ölsäureoleylester, Ölsäuredecylester, Ethyloleat, künstliches Entenbürzeldrüsenfett, und zwar jeweils einzeln oder im Gemisch.

Unter Triglyceriden werden reine oder gemischte Ester des Glycerins mit Fettsäuren der Kettenlänge $C_8$—$C_{18}$ verstanden, vorzugsweise Capryl- und/oder Caprinsäuretriglyceride.

Unter Fettsäuren werden gesättigte oder ungesättigte Fettsäuren, vorzugsweise solche mit 12—24 C-Atomen, einzeln oder im Gemisch miteinander, besonders bevorzugt Ölsäure, verstanden.

Unter Ölen im Sinne der Erfindung werden ferner verstanden: Süßmandelöl, Avocadoöl, Sesamöl, Rizinusöl, Olivenöl, Traubenkernöl Nelkenöl, Erdnußöl, Maisöl, Haselnußöl Jojobaöl, Carthamaöl und Weizenkeimöl, jeweils einzeln oder im Gemisch.

Unter *Harzen* im Sinne der vorliegenden Erfindung werden Kolophonium, dehydriertes Kolophonium, Glycerinester von dehydriertem Kolophonium, Glycerinester von Kolophoniumgummi, hydriertes Kolophonium, Glycerinester von hydriertem Kolohphonium, Pentaerythritester von hydriertem Kolophonium, Methylester von hydriertem Kolophonium, polymerisiertes Kolophonium, Glycerinester von polymerisiertem Kolophonium, Terpenharze, Cumaron/Inden-Harze, hydrierte Petroleumharze, mit Maleinsäureanhydrid modifiziertes Kolophonium und Kolophoniumderivate, $C_5$-Petroleumharze und Halbester von Styrol/Maleinsäure-Co-polymeren einzeln oder im Gemisch miteinander verstanden. Besonders bevorzugt werden Polyterpenharze aus Alpha- bzw. Beta-Pinen oder modifizierte Glycerinester der Kolophoniums. Diese Harze können je nach den erforderlichen Eigenschaften hinsichtlich der Klebrigkeit und der Haftfestigkeit auf dem Teil, an dem das resultierende Pflaster angebracht werden soll, entweder allein oder in Kombination miteinander verwendet werden.

Der Wirkstoff Etofenamat kann in einer Menge von vorzugsweise 2—20 Gew.-% in die Reservoirschicht eingearbeitet werden. Die angegebenen Gew.-% beziehen sich auf das Gesamtreservoir.

Dem Wirkstoff Etofenamat können zusätlich noch weitere Wirksubstanzen zugesetzt werden oder auch kühlende oder duftabgebende Substanzen, vorzugsweise Methylsalicylat, Glycolsalicylat, Salicylsäure, Menthol, Pfefferminzöl, Kampfer, Thymol, Acrinol, Scopolaextrakt, Chlorpeniraminmaleat, Benzylnicotinat, Capsicumextrakt, Nonylvanillylamid, Capsaicin.

Erforderlichenfalls können die erfindungsgemäßen Pflaster mit Additiven und Füllstoffen, z.B. Alterungsschutzmitteln, Antioxidatien und Verstärkungsfüllstoffen versetzt werden, soweit die gelartigen Eigenschaften nicht zerstört werden.

Bekannte Wirkstoffabgabesysteme, wie z.B. Gele, Salbengrundlagen, Pflaster geben ca. 0,5 bis 5% Wirkstoff in 7 Stunden frei. Das oben beschriebene erfindungsgemäße medizinische Pflaster dagegen setzt in 7 Stunden bis zu 33% Wirkstoff frei mit einer signifikant größeren Bioverfügbarkeit. Die erfindungsgemäßen Systeme können durch Änderung des Polymeranteils, des Schleppmittels bzw. des Harzes bezüglich ihrer Wirkstoffabgabegeschwindigkeit nahezu beliebig eingestellt werden.

Die Herstellung des etofenmathaltigen Reservoirs und des darauf basierenden Pflasters kann z.B. wie folgt durchgeführt werden: Die Pflastergrundstoffe (Polymer, Harz und Schleppmittel) werden in ein geeignetes Lösegefäß eingebracht und unter Rühren in Benzin gelöst. Es resultiert eine klare bis leicht getrübte Lösung 1. Der Wirkstoff Etofenamat wird ebenfalls in geeignetem Lösungsmittel gelöst und der Polymerlösung 1 zugesetzt.

Die so erhaltene etofenamathaltige Lösung 2 wird gleichmäßig auf silikonisiertes Papier aufgebracht und zu einem Film ausgezogen. Das beschichtete Papier mit der Plfastergrundlage wird 24 Stunden an der Luft getrocknet und dann 1 Stunde im Umlufttrockenschrank bei 40°C aufbewahrt.

Die Erfindung betrifft auch ein Verfahren zur Herstellung der erfindungsgemäßen Pflaster, das dadurch gekennzeichnet ist, daß man die Polymerkomponente, bestehend aus Polyisobutylen mit einer Molmassenverteilung $M_w/M_n$ von 1,5 bis 3,5 und einer mittleren Viskosität/Molmasse von 30 000 bis 4 000 000 g/mol, ein oder mehrere Schleppmittel und ein oder mehrere Harze in einem Lösungsmittel löst und 1 bis 30 Gew.-% Etofenamat, bezogen auf das Gemisch aus Polymerkomponente, Schleppmittel und Harz, ebenfalls in einem Lösungsmittel zur Lösung bringt, diese Lösungen vereinigt und die vereinigten Lösung gleichmäßig auf eine für den Wirkstoff Etofenamat im wesentlichen undurchlässige Folie (Deckschicht) aufträgt und zu einem Film auszieht, die beschichtete Folie (Deckschicht) zunächst bei Raumtemperatur und anschließend bei Temperaturen bis zu 50°C trocknet und gegebenenfalls die getrocknete Folie (Deckschicht) auf der beschichteten Seite mit einer für den Wirkstoff Etofenamat im wesentlichen undurchlässigen abziehbaren Schutzschicht versieht.

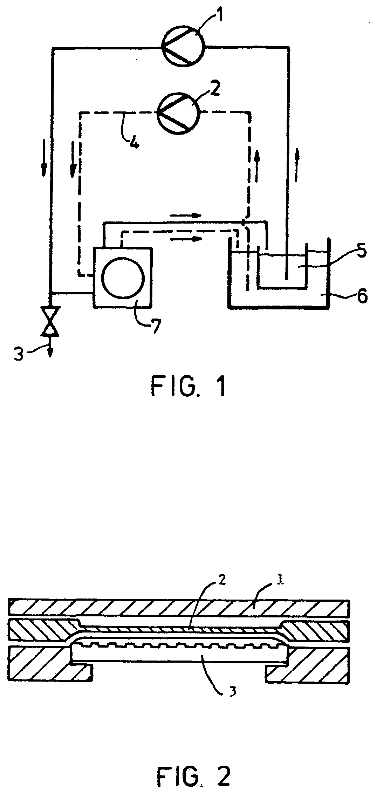

Die Bestimmungen der Wirkstoffabgaberaten erfolgten in einem Resorptionsmodell, das im folgenden und im experimentellen Teil genauer beschrieben ist. Fig. 1 zeigt ein Resorptionsmodell. Fig. 2 zeigt eine Resorptionszelle.

In Fig. 1 bedeutet 1 eine Schlauchpumpe für den Akzeptor, 2 eine Schlauchpumpe zum Temperieren, 3 die Probeentnahme, 4 den Kreislauf für die Temperierflüssigkeit, 5 das Akzeptormedium, 6 das Temperiergefäß und 7 die Resorptionszelle mit Membran.

In Fig. 2 bedeutet 1 ein undurchsichtiges Zellenmaterial, 2 eine Membrane und 3 ein Sichtfenster aus Glas, das zugleich eine Riffelplatte für das Akzeptormedium ist.

In-vitro Freisetzungsprüfung der erfindungsgemäßen Pflaster

Alle Pflaster wurden nach der gleichen Grundrezeptur mit 15% (10 bzw. 5%) Etofenamtgehalt hergestellt:

| Polymer | 50 Gew.-Teile |
| Schleppmittel | 50 Gew.-Teile |
| Harz | 10 Gew.-Teile |

(Lösungsmittel: Benzin, Hexan oder eine Mischung aus Hexan und Toluol)

Hierzu wurden alle Komponenten gelöst oder suspendiert. Als Lösungsmittel für den Wirkstoff Etofenamat wurden überwiegend Aceton und/oder Ethanol verwendet.

Zu den oben angegebenen Lösung wurde der Wirkstoff in Mengen von 17,65 Teilen (d.h. 15% der fertigen Mischung) zugegeben. Diese Lösungen bzw. Suspensionen wurden zu dünnen Folien einer Dicke von 50—150 μm verarbeitet.

Versuchsparameter:

—Akzeptormedium : Mischung aus Wasser, Ethanol, PVP, Sorbitanfettsäureester

—Volumen Akezptormedium : 200 ml

—Temperatur Akzeptormedium : 35—36°C

—Pumpleistung : 14 ml/min. (Gerätekonstante)

—Membran : als Membran wurde die in Beispiel 3 der DE-OS 3 312 735 beschriebene Folie eingesetzt

Resorptionsfläche : 28,28 cm² (Zellenkonstante)

Das Akzeptormedium wurde in einem Vorratsgefäß temperiert und über Schläuche in die Resorptionszelle umgepumpt. Die Probenentnahme erfolgte zwischen der Pumpe und den Resorptionszellen. Die Probenziehung erfolgte in festgelegten Zeitabständen. Es wurden je 6 ml Probe entnommen und spektralphotometrisch vermessen. Ein Ersatz der Akzeptorflüssigkeit erfolgte nicht, da dies eine Verdünnung der Restmenge bedeuten würde.

Berechnung der Ergebnisse

Zunächst wurde eine Eichkurve der Wirkstoffkomponente (Etofenamat) aufgenommen, mit deren Hilfe dann aus den für die Einzelproben gemessenen Extinktionswerten die Wirkstoffkonzentration (mg oder%) in den Einzelproben ermittelt wurde. Die Extinktionen wurden UV-spektroskopisch gemessen.

Zur Berechnung der "relativen Resorption" (Anteil "resorbierter" Wirkstoff am Gesamtgehalt des Pflasters in %) ist die Kenntnis der eingesetzten Wirkstoffmenge pro Pflasterfläche (28,28 cm²) nötig. Diese ist aus der Herstellung des Pflasters bekannt; die Menge des eingesetzten Pflasters ergibt sich aus der Differenz des Gewichts des ausgestanzten Pflasterstücks und dem Gewicht des gleich großen Stücks des silikonisierten Papiers. Aus dem Wirkstoffgehalt und dem Gewicht des eingesetzten Pflasters läßt sich die Menge Wirkstoff in 28,28 cm² Pflaster berechnen (Gl. 1)

Der prozentuale Anteil des "resorbierten" Wirkstoffs am Wirkstoffgehalt des Pflasters wird wie folgt berechnet:

$$\% \text{ "resorbierter" Wirkstoff bis zur Zeit } t = \frac{\text{mg Wirkstoff in Probe} \cdot V_t}{100} + M_t$$

$$M_t = \sum_{i=0}^{i=n-1} \left( V_s \cdot \frac{C_i}{100} \right)$$

$V_t$=Volumen des Akzeptormediums zur Zeit t in ml
$V_s$=Probevolumen in ml
$M_t$=entnommene Wirkstoffmenge bis zur Zeit t in mg
n=Anzahl der Proben zur Zeit t
$C_i$=Wirkstoff-Konzentration in der Probe [mg/ml]

Pflaster mit Polyisobutylen als Polymer und Etofenamat als Wirkstoff

Beispielserie A

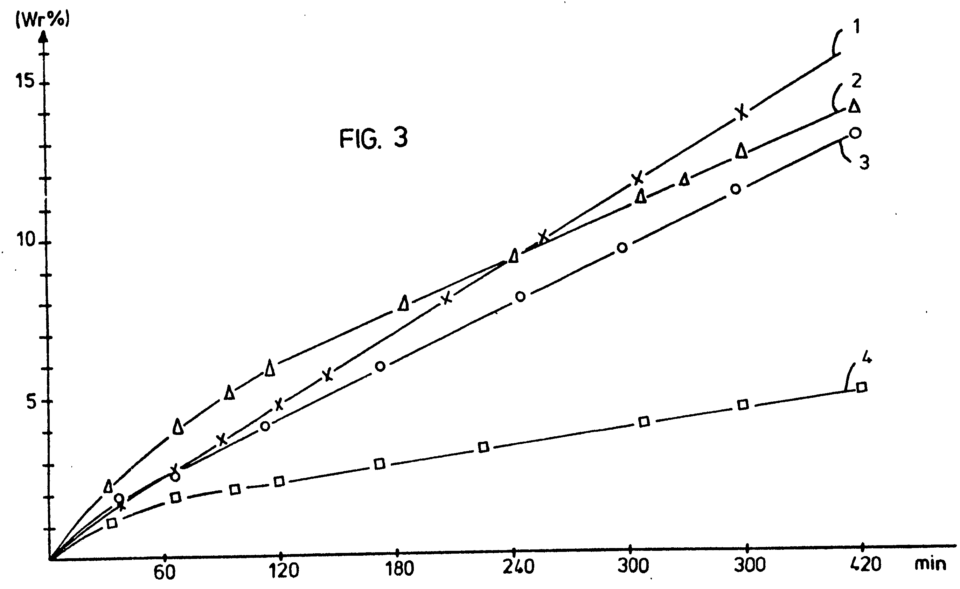

Bei dieser Versuchsreihe wurden Polyisobutylene verschiedenen Molekulargewichts als Polymer, Paraffin dünnflüssig als Schleppmittel und Colophoniumharz-Ester als Harz eingesetzt. Die genaue Zusammensetzung der Pflastergrundlagen ist in Tab. 1 angegeben. Die Herstellung erfolgte wie vorstehend beschrieben. Die Konzentration an Etofenamat beträgt 15% im fertigen, lösungsmittelfreien Pflaster. Die Freisetzungsraten sind in Figur 3 beschrieben. Wr in allen Figure 3—6 bedeutet Wirkstoffreisetzung.

Die Kurven in Fig. 3 wurden erzielt für:

|  |  |
|---|---|
| Kurve 1 | PIB MG=400 000<br>Nr. 102 in Tabelle 1 |
| Kurve 2 | PIB MG=40 000<br>Nr. 111 in Tabelle 1 |
| Kurve 3 | PIB MG=2 800 000<br>Nr. 110 in Tabelle 1 |
| Kurve 4 | PIB MG 1 270 000<br>Nr. 101 in Tabelle 1 |

Beispielserie B

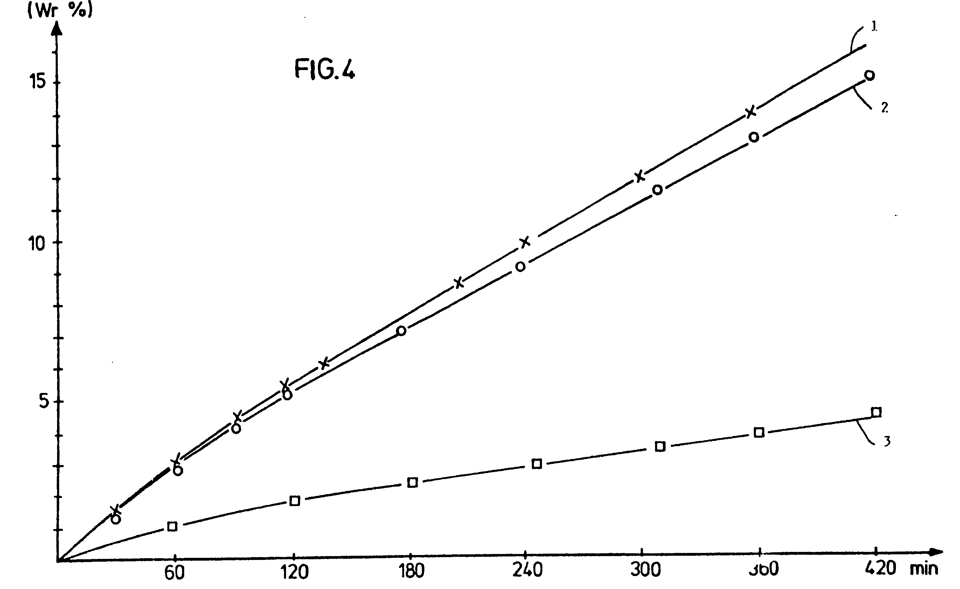

In den weiteren Versuchen wurde als Polymerkomponente stets PIB MG 400 000 eingesetzt. Bei den Versuchen wurde der Paraffinanteil von ursprünglich 50 Teilen auf 40 bzw. 60 Teile verändert (Tabelle 1). Die Anteile an Polyisobutylen und Harz wurden konstant gehalten. Die Ergebnisse sind in Fig. 4 dargestellt.

Die Kurven in Fig. 4 wurden erzielt für Paraffinanteile von:

|  |  |  |
|---|---|---|
| Kurve 1 | 50 | Nr. 102 in Tabelle 1 |
| Kurve 2 | 60 | Nr. 107 in Tabelle 1 |
| Kurve 3 | 40 | Nr. 106 in Tabelle 1 |

Beispielserie C

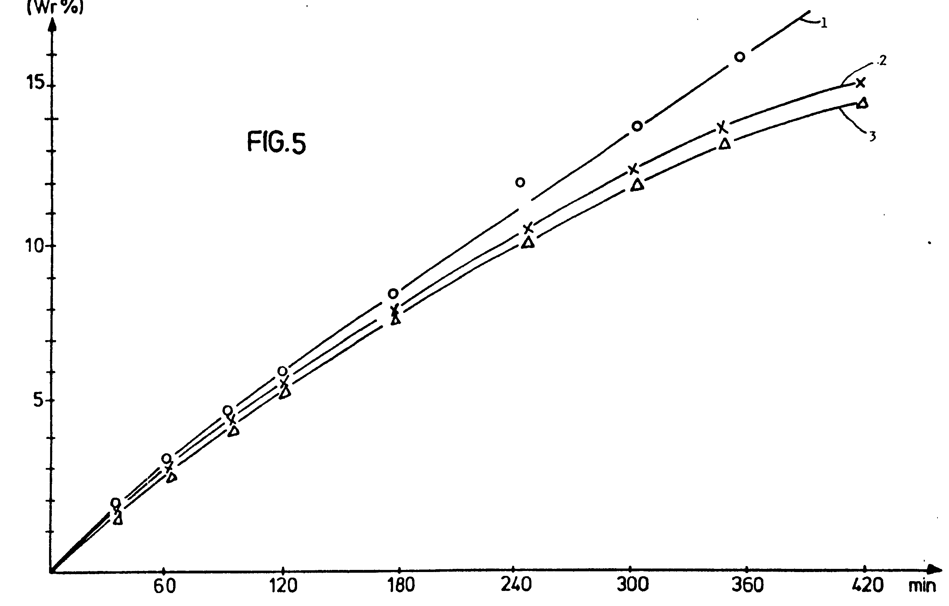

Bei konstanten Anteilen Polyisobutylen (M.G. 400 000) und Paraffin wurde der Harzanteil halbiert bzw. verdoppelt. Die Verdoppelung des Harzanteils bewirkt eine geringfügige Verbesserung der "Resorption" aus dem Plaster, während die Halbierung des Harzanteils praktisch keine Veränderung brachte (Fig. 5).

Die Kurven in Fig. 5 wurden erzielt für:

|  |  |
|---|---|
| Kurve 1 | Harzanteil 16 Teile<br>Nr. 108 in Tabelle 1 |
| Kurve 2 | Harzanteil 8 Teile<br>Nr. 107 in Tabelle 1 |
| Kurve 3 | Harzanteil 4 Teile<br>Nr. 109 in Tabelle 1 |

Beispielserie D

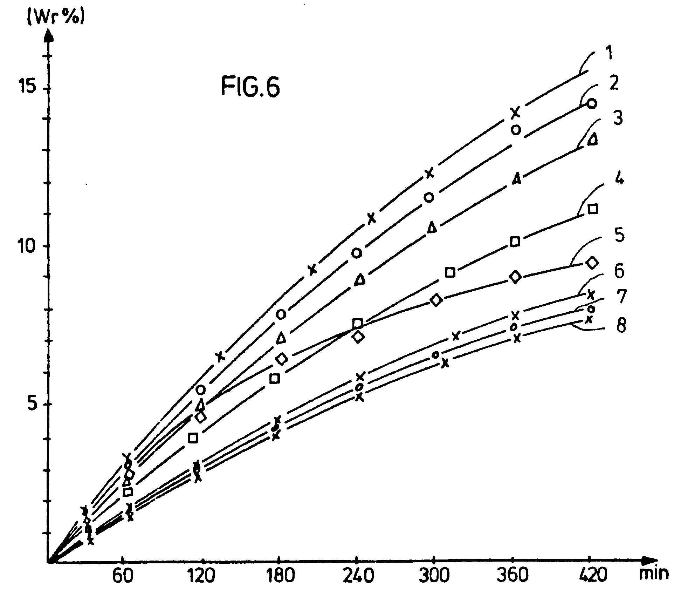

Variation des Schleppmittels (Fig. 6, Tabelle 2).

Analog Formulierung 102 wurden verschiedene Schleppmittel (statt Paraffin) bezüglich ihres Einflusses auf die Freigaberaten untersucht. Die Ergebnisse zeigt Fig. 6.

In Fig. 6 wurden die Kurven erzielt für:

# EP 0 148 391 B1

Schleppmittel

| | |
|---|---|
| Kurve 1<br>Nr. 102 in Tab. 1 | Paraffin dünnflüssig |
| Kurve 2<br>Nr. 406 in Tab. 2 | Polybutadinöl MG 1500 |
| Kurve 3<br>Nr. 405 in Tab. 2 | Ölsäuredecylester |
| Kurve 4<br>Nr. 403 in Tab. 2 | Silikonöl MG 390 |
| Kurve 5<br>Nr. 401 in Tab. 2 | Polyisobutylenöl MG 890 |
| Kurve 6<br>Nr. 407 in Tab. 2 | Polybutadinöl MG 3000 |
| Kurve 7<br>Nr. 402 in Tab. 2 | Silikonöl MG 600 |
| Kurve 8<br>Nr. 404 in Tab. 2 | Isopropylmyristat |

## TABELLE 1

| Nr. | Paraffin dünnfl./g | Colophonium-harzester/g | Polyiso-butylen M.G. | /g | Polyiso-butylen M.G. | /g | Benzin /g | Etofenamat /g | Aceton /g |
|---|---|---|---|---|---|---|---|---|---|
| 101 | 50 | 10 | 1.270.000 | 40 | — | — | 300 | 17,65 | 40 |
| 102 | 50 | 10 | 400.000 | 40 | — | — | 300 | 17,65 | 40 |
| 106 | 40 | 12 | 400.000 | 48 | — | — | 300 | 17,65 | 40 |
| 107 | 60 | 8 | 400.000 | 32 | — | — | 300 | 17,65 | 40 |
| 108 | 55 | 16 | 400.000 | 29 | — | — | 300 | 17,65 | 40 |
| 109 | 62,5 | 4 | 400.000 | 33,5 | — | — | 300 | 17,65 | 40 |
| 110 | 50 | 10 | 2.800.000 | 40 | — | — | 600 | 17,65 | 40 |
| 111 | 50 | 10 | 40.000 | 40 | — | — | 300 | 17,65 | 40 |
| 115 | 50 | 10 | 400.000 | 40 | — | — | 300 | 5,26 | 40 |

TABELLE 2

| Nr. | flüssige Komponente Art | /g | Colophonium-harzester /g | Polyisobutylen M.G. 400.000 /g | Benzin /g | Etofenamat /g | Aceton /g |
|---|---|---|---|---|---|---|---|
| 401 | Polyisobutylenöl MG 820 | 50 | 10 | 40 | 300 | 17,65 | 40 |
| 402 | Polydimethylsiloxan MG ca. 600 | 50 | 10 | 40 | 300 | 17,65 | 40 |
| 403 | Polydimethylsiloxan MG 390 | 50 | 10 | 40 | 300 | 17,65 | 40 |
| 404 | Isopropylmyristat | 50 | 10 | 40 | 300 | 17,65 | 40 |
| 405 | Ölsäuredecylester | 50 | 10 | 40 | 300 | 17,65 | 40 |
| 406 | Polybutadien Öl MG 1500 | 50 | 10 | 40 | 300 | 17,65 | 40 |
| 407 | Polybutadien Öl MG 3000 | 50 | 10 | 40 | 300 | 17,65 | 40 |

Herstellungsbeschreibung

Die erfindungsgemäßen medizinischen Pflaster wurden folgendermaßen hergestellt: Das Gemisch aus Polymer, Harz und Schleppmittel wurde in einem Z-Kneter bei einer Temperatur von 120 bis 150°C vorgeknetet. Wenn die Masse eine homogene Schmelze darstellte, wurde unter Stickstoffbegasung der Wirkstoff homogen eingearbeitet. Die wirkstoffhaltige Schmelze wurde auf Trägerfolie aufgetragen (Kneter).

Die erfindungsgemäßen medizinischen Pflaster wurden auch in einem Lösungsmittelgemisch aufgelöst, auf die Trägerfolie aufgetragen und anschließend getrocknet (Lösung).

Beispiel 1 (Lösung)

| | |
|---|---|
| Polyisobutylen M.G. 400.000 | 40,00 g |
| Paraffinöl dünnflüssig | 50,00 g |
| Colophoniumharzester | 10,00 g |
| Etofenamat | 17,65 g |

Freisetzung: nach 6 Std. ca. 15%

Beispiel 2 (Lösung)

| | |
|---|---|
| Polyisobutylen M.G. 40.000 | 40,00 g |
| Paraffinöl dünnflüssig | 50,00 g |
| Colophoniumharzester | 10,00 g |
| Etofenamat | 17,65 g |

Freisetzung: nach 6 Std. ca. 14%

Beispiel 3 (Kneter)

| | |
|---|---|
| Polyisobutylen M.G. 400.000 | 40,00 g |
| Polybutadienöl M.G. 1500 | 50,00 g |
| Colophoniumharzester | 10,00 g |
| Etofenamat | 17,65 g |

Freisetzung: nach 6 Std. ca. 14%

Beispiel 4 (Kneter)

| | |
|---|---|
| Polyisobutylen M.G. 400.000 | 40,00 g |
| Ölsäuredecylester | 50,00 g |
| Colophoniumharzester | 10,00 g |
| Etofenamat | 17,65 g |

Freisetzung: nach 6 Std. ca. 13%

Beispiel 5 (Kneter)

| | |
|---|---|
| Polyisobutylen M.G. 400.000 | 40,00 g |
| Silikonöl | 50,00 g |
| Colophoniumharzester | 10,00 g |
| Etofenamat | 17,65 g |

Freisetzung: nach 6 Std. ca. 9,5 %

Beispiel 6 (Lösung)

| | |
|---|---|
| Polyisobutylen M.G. 400.000 | 32,00 g |
| Paraffin dünnflüssig | 60,00 g |
| Colphoniumharzester | 8,00 g |
| Etofenamat | 17,65 g |

Freisetzung: nach 6 Std. ca. 13,5 %

Beispiel 7 (Lösung)

| | |
|---|---|
| Polyisobutylen M.G. 400.000 | 29,00 g |
| Paraffin dünnflüssig | 55,00 g |
| Colophoniumharzester | 16,00 g |
| Etofenamat | 17,65 g |

Freisetzung: nach 6 Std. ca. 15,5 %

Beispiel 8 (Lösung)

| | |
|---|---|
| Polyisobutylen M.G. 400.000 | 33,50 g |
| Paraffin dünnflüssig | 62,50 g |
| Colophoniumharzester | 4,00 g |
| Etofenamat | 17,65 g |

Freisetzung: nach 6 Std. ca. 14,5 %

Beispiel 9 (Lösung)

| | |
|---|---|
| Polyisobutylen M.G. 400.000 | 40,00 g |
| Paraffin dünnflüssig | 50,00 g |
| Colophoniumharzester | 10,00 g |
| Etofenamat | 5,26 g |

Freisetzung: nach 6 Std. ca. 32,5%

Beispiel 10 (Lösung)

| | |
|---|---|
| Polyisobutylen M.G. 400.000 | 40,00 g |
| Polybutandienöl M.G. 1500 | 50,00 g |
| Polyterpenharz aus α-Pinen | 10,00 g |
| Etofenamat | 17,65 g |

Freisetzung: nach 6 Std. ca. 16 %

Beispiel 11 (Lösung)

| | |
|---|---|
| Polyisobutylen M.G. 400.000 | 40,00 g |
| Paraffinöl dünnflüssig | 50,00 g |
| Polyterpenharz aus β-Pinen | 10,00 g |
| Etofenamat | 17,65 g |

Freisetzung: nach 6 Std. ca. 16,5 %

Beispiel 12 (Kneter)

| | |
|---|---|
| Polyisobutylen M.G. 400.000 | 40,00 g |
| Polybutadienöl M.G. 1500 | 50,00 g |
| Modifizierter glycerinester des Colophoniums | 10,00 g |
| Etofenamat | 5,26 g |

Freisetzung: nach 6 Std. ca. 33 %

**Patentansprüche**

1. Medizinisches Pflaster zur transdermalen Verabreichung von Antiphlogistika, bestehend aus einer Deckschicht, einer abziehbaren Schutzschicht und einer den Wirkstoff, Schleppmittel und Harz enthaltenden einlagigen Reservoirschicht auf Basis von Polymeren, dadurch gekennzeichnet, daß die Reservoirschicht als Polymere Polyisobutylene mit einer Molmassen-Verteilung $M_w/M_n$ von 1,5 bis 3,5 und eine mittlere Viskosität/Molmasse von 30 000 bis 4 000 000 g/mol enthält und 1 bis 30 Gew.-% des Wirkstoffs Etofenamat enthält.

2. Medizinisches Pflaster nach Anspruch 1, dadurch gekennzeichnet, daß es in der Reservoirschicht 5 bis 15 Gew.-% Etofenamat enthält.

3. Medizinisches Pflaster nach den Ansprüchen 1 und 2, dadurch gekennzeichnet, daß die Reservoirschicht neben dem Etofenamat 30 bis 60 Gew.-% Polyisobutylen als Polymer, 30 bis 60 Gew.-% Schleppmittel und 2 bis 40 Gew.-% klebrig machendes Harz enthält, wobei sich die drei Komponenten zu 100 Gew.-% ergänzen.

4. Medizinisches Pflaster nach den Ansprüchen 1 bis 3, dadurch gekennzeichnet, daß Polyisobutylene mit einer mittleren Viskosität von 50 000 bis 1 000 000 g/mol eingesetzt werden.

5. Medizinische Pflaster nach den Ansprüchen 1 bis 4, dadurch gekennzeichnet, daß Polyisobutylene mit einer mittleren Viskosität von 80 000 bis 500 000 g/mol eingesetzt werden.

6. Verfahren zur Herstellung von medizinischen Pflastern nach den Ansprüchen 1 bis 5, dadurch gekennzeichnet, daß man Polyisobutylen Molmassenverteilung $M_w/M_n$ von 1,5 bis 3,5 und mit einer mittleren Viskosität/Molmasse von 30 000 bis 4 000 000 g/mol, 1 oder mehrere Schleppmittel und 1 oder mehrere Harze in einem Lösungsmittel löst, dann 1 bis 30 Gew.-% Etofenamat, bezogen auf das Gemisch aus Polymerkomponente, Schleppmittel und Harz, ebenfalls in einem Lösungsmittel zur Lösung bringt, diese Lösungen vereinigt und die vereinigten Lösungen gleichmäßig auf eine, für den Wirkstoff im wesentlichen undurchlässige Folie (Deckschicht) aufträgt und zu einem Film auszieht, die beschichtete Folie (Deckshicht) zunächst bei Raumtemperatur und anschließend bei Temperaturen bis zu 50°C trocknet und gegebenenfalls die getrocknete Folie (Deckschicht) auf der beschichteten Seite mit einer für den Wirkstoff im wesentlichen undurchlässigen abziehbaren Schutzschicht versieht.

**Revendications**

1. Emplâtre médicinal pour l'administration transdermique d'antiphlogistiques, consistant en une couche de couverture, une couche de protection éliminable et une couche réservoir à base de polymères, en une seule épaisseur, qui contient la substance active, les agents d'entraînement et les résines, caractérisé en ce que la couche réservoir contient en tant que polymères des polyisobutylènes à une répartition des masses moléculaires $M_w/M_n$ de 1,5 à 3,5 et un rapport viscosité moyenne/masse moléculaire de 30 000 à 4 000 000 de g/mole, et de 1 à 30% en poids de la substance active Etofenamat.

2. Emplâtre médicinal selon la revendication 1, caractérisé en ce qu'il contient dans la couche réservoir de 5 à 15% en poids d'Etofenamat.

3. Emplâtre médicinal selon les revendications 1 à 2, caractérisé en ce que la couche réservoir contient, un plus de l'Etofenamat, de 30 à 60% en poids d'un polyisobutylène constituant le polymère, de 30 à 60% en poids d'agents d'entraînement et de 2 à 40% en poids d'une résine collante, les proportions des trois composants se complétant à 100% en poids.

4. Emplâtre médicinal selon les revendications 1 à 3, caractérisé en ce que l'on utilise des polyisobutylènes ayant une viscosité moyenne de 50 000 à 1 000 000 de g/mole.

5. Emplâtre médicinal selon les revendications 1 à 4, caractérisé en ce que l'on utilise des

# EP 0 148 391 B1

polyisobutylènes ayant une viscosité moyenne de 80 000 à 500 000 g/mole.

6. Procédé de préparation des emplâtres médicinaux selon les revendications 1 à 5, caractérisé en ce que l'on dissout dans un solvant du polyisobutylène à une répartition des masses moléculaires $M_w/M_n$ de 1,5 à 3,5 et un rapport viscosité moyenne/masse moléculaire de 30 000 à 4 000 000 de g/mole, un ou plusieurs agents d'entraînement et une ou plusieurs résines, on dissout ensuite 1 à 30% en poids d'Etofenamat, par rapport au mélange du composant polymère, des agents d'entraînement et de la résine, également dans un solvant, on combine ces solutions et on applique uniformément la solution combinée sur une feuille essentiellement imperméable pour la substance active (couche de couverture) et on étire en une pellicule, on sèche la feuille revêtue (couche de couverture) d'abord à la température ambiante puis à des températures allant jusqu'à 50°C et, les cas échéant, on applique sur la feuille séchée (couche de couverture), sur la face revêtue, une couche de protection éliminable essentiellement imperméable pour la substance active.

## Claims

1. Medical plaster for transdermal administration of antiphlogistics, containing a covering layer, a protective layer which can be pulled off and a single-layered reservoir layer which contains the active compound, entraining agents and resins and is based on polymers, characterized in that the reservoir layer contains, as polymers, polyisobutylenes having a molecular weight distribution $M_w/M_n$ of 1.5 to 3.5 and a viscosity average of the molecular weight of 30,000 to 4,000,000 g/mol, and contains 1 to 30% by weight of the active compound etophenamate.

2. Medical plaster according to Claim 1, characterized in that it contains 5 to 15% by weight of etophenamate in the reservoir layer.

3. Medical plaster according to Claims 1 and 2, characterized in that the reservoir layer contains, in addition to the etophenamate, 30 to 60% by weight of polyisobutylene, as the polymer, 30 to 60% by weight of entraining agent and 2 to 40% by weight of tackifying resin, the three components making up 100% by weight.

4. Medical plaster according to Claims 1 to 3, characterized in that polyisobutylenes having a viscosity average of 50,000 to 1,000,000 g/mol are employed.

5. Medical plaster according to Claims 1 to 4, characterized in that polyisobutylenes having a viscosity average of 80,000 to 500,000 g/mol are employed.

6. Process for the production of medical plasters according to Claims 1 to 5, characterized in that polyisobutylene of molecular weight distribution $M_w/M_n$ of 1.5 to 3.5 and having a viscosity average of the molecular weight of 30,000 to 4,000,000 g/mol, one or more entraining agents and one or more resins are dissolved in a solvent, 1 to 30% by weight of etophenamate, based on a mixture of polymer components, entraining agent and resin, is likewise dissolved in a solvent, these solutions are combined and the combined solutions are applied uniformly to a foil (covering layer) which is essentially impermeable to the active compound and are drawn out to a film, the second foil (covering layer) is dried first at room temperature and then at temperatures up to 50°C and if appropriate the dried foil (covering layer) is provided, on the coated side, with a protective layer which is essentially impermeable to the active compound and can be pulled off.

10

FIG. 1

FIG. 2

FIG. 3

FIG.4

FIG.5

(Wr %)

EP 0 148 391 B1

FIG.6